# EUROPEAN PATENT SPECIFICATION

(11) **EP 2 461 705 B1**
(45) Date of publication and mention of the grant of the patent: **04.12.2013**
(21) Application number: 09787836.7
(22) Date of filing: 06.08.2009
(51) Int. Cl.: A23L 1/30, A61K 35/74, C12N 1/04

(54) **NUTRACEUTICAL COMPOSITIONS CONTAINING PROBIOTICS IN AN OILY VEHICLE**
NUTRAZEUTISCHE ZUSAMMENSETZUNGEN MIT PROBIOTIKA IN EINEM ÖLIGEN TRÄGERSTOFF
COMPOSITIONS D'ALICAMENTS CONTENANT DES PRODUITS PROBIOTIQUES DANS UN VÉHICULE HUILEUX

(43) Date of publication of application: 13.06.2012
(73) Proprietor: Labomar S.r.l., 31036 Istrana (TV) (IT)
(72) Inventor: FRATTER, Andrea, 30030 Olmo di Martellago (VE) (IT); BERTIN, Walter, 31036 Istrana (TV) (IT)
(74) Representative: Asensio, Raffaella Consuelo
(86) International application number: PCT/IT2009/000373
(87) International publication number: WO 2011/016070

(56) References cited:
- US-A- 4 518 696
- US-A1- 2004 001 817
- US-A1- 2005 106 132

## Description

### Field of the invention

The present invention concerns nutraceutical compositions that contain probiotics, in particular lactobacilli.

### Background of the invention

The term "probiotics" identifies beneficial microorganisms, be they bacteria (for example lactobacilli and bifidobacteria) or fungi (for example yeasts), which contribute to maintaining a correct composition of the intestinal microflora in mammals. Following stress, pathological states or administering of drugs, the composition of the microflora can vary, with consequent alteration of the functionality of the gastrointestinal tract. It has been demonstrated that administering probiotics not only helps restore normal physiological functions, but also helps to prevent diarrheal forms caused by pathogenic bacteria, infections of the urogenital apparatus and atopic diseases.

One of the problems associated with administering probiotics consists of the fact that their vitality decreases if the process for preparing the administration forms is carried out in uncontrolled conditions, for example if the temperature increases excessively (usually above 40 °C) or if the humidity during the course of the process is too high. Moreover, the content of vital microorganisms in formulations can decrease over time when stored in less than optimal conditions (high air humidity and temperature).

A further problem associated with the administering of probiotics consists of the fact that their vitality substantially decreases in the stomach due to the action of the gastric juices and the low pH value, whereas it is necessary for them to reach the intestine in sufficient number to carry out their beneficial effect; therefore, it is necessary to provide functional foods or compositions that are also able to protect such microorganisms in the gastric environment.

Several nutraceutical compositions and functional foods containing probiotics are currently on the market. The problem of controlling the water content has been solved, on the one hand, by preparing solid formulations, like for example microcapsules, and on the other hand by replacing the water in liquid formulations with oily vehicles. US 4,518,696 discloses, for example, liquid compositions containing probiotics, in particular lactobacilli, dispersed in sunflower seed oil in the presence of silica ("fumed silica"). However, it is known that even oily solvents contain a certain percentage of water, which can impair the stability of the microorganisms. Moreover, oil-based formulations may not be easily redispersed in water, thus making them difficult or unpleasant to take, particularly for children.

Oil-based formulations containing biological material, in particular probiotic microorganisms, are for example described in WO 2009/061222; in such formulations the stabilization effect is achieved by coating the biological material with an oil and a natural polymer. This application also teaches to optionally add a desiccant, of which silica is one, in variable quantity, to obtain a denser consistency, which can thus be subjected to extrusion or compression to obtain tablets, granules, pills or pellets.

### Description of the invention

The object of the present invention are nutraceutical compositions containing probiotics in an oily vehicle, characterized in that they contain a sorbitan ester, an ascorbyl palmitate salt and silica.

For the purposes of the present invention, the term "probiotic" identifies probiotic microorganisms selected, from bacteria and fungi, including yeasts, be they natural or genetically modified. It should also be understood that the formulations of the invention can contain microorganisms belonging to the same strain, or else microorganisms belonging to different strains. Preferably, the compositions of the invention concern bacteria, like lactobacilli or bifidobacteria; specific examples of the former are *Lactobacillus Acidophilus, Lactobacillus Casei, Lactobacillus Casei Immunitas, Lactobacillus Acidophilus, Lactobacillus Reuteri, Lactobacillus Bulgaricus, Lactobacillus Sporogenes, Lactobacillus spp,* whereas specific examples of the latter are *Bifidobacterium Bifidus, Bifidobacterium lactis, Bifidobacterium longum, Bifidobacterium brevis, Bifidobacterium actiregularis.* According to one particular embodiment of the invention, the lattobacillus is *Lactobacillus rhamnosus* LGG ATCC 53103 (disclosed in US 4,839,281), which produces an antimicrobial substance acting against various bacteria of the intestinal microflora and which has been proven effective in the treatment of Rotavirus diarrhea, travelers' diarrhea and *Clostridium difficile* diarrhea.

The term "oily vehicle" identifies one or more lipids of the triglyceride type with different carbon chain length, usually between 6 and 18 carbon atoms; examples of such lipids are coconut oil, wheat germ oil, sunflower seed oil, olive oil and triglycerides derived from them; preferably, medium carbon chain C₈-C₁₀ triglycerides are used, like caprylic/capric triglyceride derived from coconut oil. Commercially available products based on these lipids are produced by Cognis with the trade names Migliol^{®} 810, Migliol^{®} 812, Delios^{®} C, Delios^{®} V, Delios^{®} S o Cetiol^{®} LC.

The term "sorbitan ester" identifies a sorbitan ester with high HLB, usually between 10 and 20; examples of sorbitan esters with high HLB value are: polyoxyethylen-(20)-sorbitan monolaurate (polysorbate 20); polyoxyethylen- (20)-sorbitan monopalmitate (polysorbate 40); polyoxyethylen- (20)-sorbitan monostearate (polysorbate 60); polyoxyethylen- (20)-sorbitan tristearate (polysorbate 65); polyoxyethylen- (20)-sorbitan monooleate (polysorbate 80) and polyoxyethylen-sorbitan trioleate (polysorbate 85). According to a preferred embodiment of the invention, the sorbitan ester is selected from polysorbate 60 and polysorbate 80; the latter is particularly advantageous from the point of view of formulation since it is liquid.

Ascorbyl palmitate is the ester of L-ascorbic acid with palmitic acid (C 16) and it is widely used in the cosmetics and food industries as antioxidant and stabilizing agent for emulsified systems, in particular as anti-oxidant for preventing rancidicity in fats (E304). In the cosmetics industry it is used as a skin-absorbable form of L-ascorbic acid. Thanks to the amphiphilic structure given to it by the hydrophilic portion of the L-ascorbic acid and by the lipophilic portion of the palmitic acid, ASP behaves like a surfactant. It is *per sé* not very soluble in water, but at concentrations of between 0.5 and 5.0% (p/p with respect to the aqueous phase), in particular between 2 and 3% p/p, and at high temperatures, it dissolves and forms a compact white coagel by cooling. However, since ASP possesses two acid hydroxyls (enediol) it is possible to obtain clear solutions even under cold conditions by addition of a base, thanks to the formation of an ASP salt, which is advantageous for the purposes of obtaining emulsions of thermolabile active ingredients or substances, like probiotics. In the compositions of the invention an ascorbic acid salt is used with a basic amino acid like L-arginine or L-lysine or with an alkaline reacting peptide, for example L-carnosine. According to one particular embodiment of the invention, ascorbic acid is salified with L-arginine.

For the preparation of the compositions of the invention probiotic microorganisms in dry state are used, in which the water content has already been reduced with known methods, for example through drying or freeze-drying processes known to the person skilled in the art, and in which the cell concentration is between 100 x 10⁹ and 500 x 10⁹ unit forming colonies (ufc)/gr, preferably between 300 x 10⁹ and 400 x 10⁹ ufc/gr. Usually, the compositions of the invention contain a percentage by weight of microorganism in powder form of between 2 and 10%, preferably between 3 and 5% with respect to the oily vehicle. In the present description, the percentages of the individual ingredients are expressed by weight with respect to the weight of the oily vehicle. The sorbitan ester content is between 20 and 50%; the ascorbic acid ester content is between 3 and 10%, preferably between 5 and 8%; the silica content is between 0.5 and 5%, preferably between 1 and 3%, whereas the content of organic base, in particular of L-arginine, varies between 0.05 and 3%, preferably between 1 and 2%.

Moreover, the compositions according to the invention can also contain polyols, in a quantity of between 0.2 and 1.0%, to give a sweet flavor. Examples of suitable polyols are sorbitol, fructose and sucralose, preferably sucralose, in a quantity of between 0.010 and 0.5%.

The compositions according to the invention can be prepared with a process that comprises the following steps:
a) heating the oily vehicle to a temperature of between 50 and 80°C;
b) addition to the heated oily vehicle of the sorbitan ester, continuously stirring until a clear solution is obtained;
c) addition of silica to the solution obtained in step b), continuously stirring until a clear solution is obtained;
d) cooling to room temperature of the solution obtained in step c);
e) addition of the base, the polyol and the probiotic to the cooled solution, continuously stirring the system; preferably, these ingredients are added in succession. Where stirring is necessary, mechanical stirring is usually employed.

The compositions thus obtained can be formulated, together with possible physiologically acceptable excipients, in a way suitable for oral administering. In particular, the compositions can be packaged in multidose bottles equipped with droplet-counters, so that the user can take the suitable dose and mix it with water or with another aqueous formulation containing excipients. According to a particularly advantageous embodiment, the composition can be packaged in the form of small single-dose bottles, possibly in combination with a separate container containing an aqueous solution containing the excipients, to be mixed at the moment of administering. Therefore, the present invention also comprises a kit consisting of the composition of the invention and of an aqueous vehicle containing physiologically acceptable excipients. Moreover, the composition of the invention can be formulated in the form of soft capsules to be taken with water. These pharmaceutical formulations can be prepared with excipients and techniques known to the person skilled in the art, for example as described in Remington, "The Science and Practice of Pharmacy" (Lippincott Williams and Wilkins, 21a ed.).

The compositions of the invention are advantageous since the association between sorbitan esters and an ascorbyl palmitate salt (ASP) forms a surfactant mixture that, after the addition of an aqueous phase, allows the formation of extemporaneous emulsions by simple cold stirring. The presence of silica at the same time makes it possible to dehydrate and thicken the oily vehicle. Stability tests carried out on formulations prepared in pilot scale have demonstrated that after 90 days, both at a temperature of 25°C and of 4°C, the vitality of the strains remains unchanged. A further advantage consists of the fact that the preparation process is simple and cost-effective from the industrial point of view, since it essentially consists of mixing steps that do not require expensive apparatuses.

The invention will now be illustrated in greater detail in the following example.

### EXAMPLE

### Composition 1

16.5 gr of Delios S were weighed in a clean and dry glass beaker, 10 gr of Veremul T 60 and 1.5 gr of ascorbyl palmitate were added, after which the mixture was heated to 50°C until a clear phase was obtained without undissolved aggregates (ASP). Under continuous stirring and heating 0.6 gr of silica were added, checking that the phase thickens while remaining clear. Once the ingredients had been added, the mixture was cooled in cold water bain-marie and, once a temperature below 30°C has been reached, 0.3 gr of arginine and 0.1 gr of sucralose were added, keeping under vigorous mechanical stirring; finally, 1 gr of LGG ferments (350 x 10⁹ UFC/gr) was added, covering the beaker with aluminum until a clear dispersion was obtained, which was then packaged in 10 and 6 gram bottles.

**Single-dose 10 gr bottles**

| **Ingredient** | **Quantity (gr** |
|---|---|
| Delios S (triglycerides of caprylic/capric acid) | 5.5000 |
| Veremul T 60 (polysorbate 60) | 3.3333 |
| Ascorbyl Palmitate | 0.5000 |
| Silica | 0.2000 |
| Arginine base | 0.1000 |
| Sucralose | 0.0020 |
| Lactic Ferments (LGG Strain, 350 x 10⁹ ufc/g) | 0.3333 |
| Total | 10 gr |

**Single-dose 6 gr bottles**

| **Ingredient** | **Quantity (gr** |
|---|---|
| Delios S (triglycerides of caprylic/capric acid) | 3.300 |
| Veremul T 60 (polysorbate 60) | 2.000 |
| Ascorbyl Palmitate | 0.300 |
| Silica | 0.120 |
| Arginine base | 0.060 |
| Sucralose | 0.020 |
| Lactic Ferments (LGG Strain, 350 x 10⁹ ufc/g) | 0.200 |
| Total | **6.000** |

### Formulation 2

Proceeding as described for formulation 1, but using Veremul T 80 instead of Veremul T 60, single-dose 6 and 10 gr bottles were prepared.

## Claims

1. Nutraceutical compositions based on probiotic ferments in an oily vehicle, **characterized in that** they contain an ascorbyl palmitate salt with a basic amino acid or with an alkaline reacting peptide, a sorbitan ester and silica.

2. Compositions according to claim 1 wherein the oily vehicle is selected from one or more lipids of the triglyceride type with carbon chain length of between 6 and 18 carbon atoms.

3. Compositions according to claim 2 wherein the lipid is selected from a triglyceride with a carbon chain of between 8 and 10 carbon atoms.

4. Compositions according to claim 2) or 3) wherein the lipid is selected from one or more coconut oils, wheat germ oil, sunflower seed oil and olive oil.

5. Compositions according to claim 4) wherein the lipid is caprylic/capric triglyceride derived from coconut oil.

6. Compositions according to any one of claims 1) to 5) wherein the basic amino acid is selected from L-arginine and L-lysine and the basic reacting peptide is L-carnosine.

7. Compositions according to claim 6) wherein the basic amino acid is L-arginine.

8. Compositions according to any one of claims 1) to 7) wherein the sorbitan ester is selected from polyoxyethylen- (20)-sorbitan monolaurate (polysorbate 20); polyoxyethylen- (20)-sorbitan monopalmitate (polysorbate 40); polyoxyethylen- (20)-sorbitan monostearate (polysorbate 60); polyoxyethylen- (20)-sorbitan tristearate (polysorbate 65); polyoxyethylen-(20)-sorbitan monooleate (polysorbate 80) and polyoxyethylen-sorbitan trioleate (polysorbate 85).

9. Compositions according to claim 8) wherein the sorbitan ester is selected from polyoxyethylen- (20)-sorbitan monostearate (polysorbate 60) and polyoxyethylensorbitan monooleate (polysorbate 80).

10. Compositions according to any one of claims 1) to 9) in droplet or soft capsule form.

11. Administering kit consisting of the composition of any one of claims 1) to 9) and an aqueous vehicle containing pharmaceutically acceptable excipients.

12. Process for preparing a composition according to any one of claims 1) to 9), comprising the following steps:
a) heating the oily vehicle to a temperature of between 50 and 80°C;
b) addition of the sorbitan ester to the heated oily vehicle, continuously stirring until a clear solution is obtained;
c) addition of silica to the solution obtained in step b), continuously stirring until a clear solution is obtained;
d) cooling to room temperature of the solution obtained in step c);
e) addition of the base, the polyol and the probiotic to the cooled solution, continuously stirring the system.

## Patentansprüche

1. Nutrazeutische Zusammensetzungen, die auf probiotischen Fermenten in einem öligen Trägerstoff basieren, **dadurch gekennzeichnet, dass** sie ein Ascorbylpalmitatsalz mit einer basischen Aminosäure oder mit einem alkalisch reagierenden Peptid, ein Sorbitanester und Silica enthalten.

2. Zusammensetzungen nach Anspruch 1, wobei der ölige Trägerstoff aus einem oder mehreren Lipiden vom Triglyceridtyp mit einer Kohlenstoffkettenlänge von 6 bis 18 Kohlenstoffatomen ausgewählt ist.

3. Zusammensetzungen nach Anspruch 2, wobei das Lipid aus einem Triglycerid mit einer Kohlenstoffkette von 8 bis 10 Kohlenstoffatomen ausgewählt ist.

4. Zusammensetzungen nach Anspruch 2 oder 3, wobei das Lipid aus einem oder mehreren von Kokosnussöl, Weizenkeimöl, Sonnenblumenkernöl und Olivenöl ausgewählt ist.

5. Zusammensetzungen nach Anspruch 4, wobei das Lipid ein aus Kokosnussöl abgeleitetes Capryl/Caprin-Triglycerid ist.

6. Zusammensetzungen nach einem der Ansprüche 1 bis 5, wobei die basische Aminosäure aus L-Arginin und L-Lysin ausgewählt ist und das alkalisch reagierende Peptid L-Carnosin ist.

7. Zusammensetzungen nach Anspruch 6, wobei die basische Aminosäure L-Arginin ist.

8. Zusammensetzungen nach einem der Ansprüche 1 bis 7, wobei der Sorbitanester aus Polyoxyethylen-(20)-sorbitan-monolaurat (Polysorbat 20), Polyoxyethylen-(20)-sorbitan-monopalmitat (Polysorbat 40), Polyoxyethylen-(20)-sorbitan-monostearat (Polysorbat 60), Polyoxyethylen-(20)-sorbitan-tristearat (Polysorbat 65), Polyoxyethylen-(20)-sorbitan-monooleat (Polysorbat 80) und Polyoxyethylen-sorbitan-trioleat (Polysorbat 85) ausgewählt ist.

9. Zusammensetzungen nach Anspruch 8, wobei der Sorbitanester aus Polyoxyethylen-(20)-sorbitan-monostearat (Polysorbat 60) und Polyoxyethylen-sorbitan-monooleat (Polysorbat 80) ausgewählt ist.

10. Zusammensetzungen nach einem der Ansprüche 1 bis 9 in Tropfen- oder Softkapselform.

11. Verabreichungsset, bestehend aus der Zusammensetzung nach einem der Ansprüche 1 bis 9 und einem wässrigen Trägerstoff, der pharmazeutisch akzeptable Hilfsstoffe enthält.

12. Verfahren zum Zubereiten einer Zusammensetzung nach einem der Ansprüche 1 bis 9, umfassend die folgenden Schritte:
a) Erwärmen des öligen Trägerstoffs auf eine Temperatur von 50 bis 80 °C,
b) Hinzufügen des Sorbitanesters zu dem erwärmten öligen Trägerstoff, kontinuierlich Rühren, bis eine klare Lösung erhalten wird,
c) Hinzufügen von Silica zu der in Schritt b) erhaltenen Lösung, kontinuierlich Rühren, bis eine klare Lösung erhalten wird,
d) Abkühlen der in Schritt c) erhaltenen Lösung auf Raumtemperatur,
e) Hinzufügen der Base, des Polyols und des Probiotikums zu der abgekühlten Lösung, kontinuierlich Rühren des Systems.

## Revendications

1. Compositions nutraceutiques basées sur des ferments probiotiques dans un véhicule huileux, **caractérisées par le fait qu'**elles contiennent un sel de palmitate d'ascorbyle avec un acide aminé basique ou avec un peptide réagissant de manière alcaline, un ester de sorbitan et de la silice.

2. Compositions selon la revendication 1, dans lesquelles ledit véhicule huileux est choisi parmi un ou plusieurs lipides du type triglycéride avec une longueur de chaîne de carbone comprise entre 6 et 18 atomes de carbone.

3. Compositions selon la revendication 2, dans lesquelles le lipide est choisi parmi un triglycéride avec une chaîne de carbone de 8 à 10 atomes de carbone.

4. Compositions selon la revendication 2 ou 3, dans lesquelles le lipide est choisi parmi une ou plusieurs des huile de coco, huile de germe de blé, huile de tournesol et huile d'olive.

5. Compositions selon la revendication 4, dans lesquelles le lipide est un triglycéride caprylique/caprique dérivé de l'huile de coco.

6. Compositions selon l'une quelconque des revendications 1 à 5, dans lesquelles ledit acide aminé basique est choisi parmi la L-arginine et la L-lysine et ledit peptide réagissant de manière alcaline est la L-carnosine.

7. Compositions selon la revendication 6, dans lesquelles ledit acide aminé basique est la L-arginine.

8. Compositions selon l'une quelconque des revendications 1 à 7, dans lesquelles ledit ester de sorbitan est choisi parmi le monolaurate de polyoxyéthylène (20) sorbitane (polysorbate 20), le monopalmitate de polyoxyéthylène (20) sorbitane (polysorbate 40), le monostéarate de polyoxyéthylène (20) sorbitane (polysorbate 60), le tristéarate de polyoxyéthylène (20) sorbitane (polysorbate 65), le monooléate de polyoxyéthylène (20) sorbitane (polysorbate 80) et le trioléate de polyoxyéthylène sorbitane (polysorbate 85).

9. Compositions selon la revendication 8, dans lesquelles ledit ester de sorbitan est choisi parmi le monostéarate de polyoxyéthylène (20) sorbitane (polysorbate 60) et le monooléate de polyoxyéthylène sorbitane (polysorbate 80).

10. Compositions selon l'une quelconque des revendications 1 à 9, sous forme de gouttelettes ou de capsules molles.

11. Kit d'administration, se composant de la composition selon l'une quelconque des revendications 1 à 9 et d'un véhicule aqueux contenant des excipients pharmaceutiquement acceptables.

12. Procédé de préparation d'une composition selon l'une quelconque des revendications 1 à 9, comprenant les étapes suivantes :
a) chauffer le véhicule huileux à une température comprise entre 50 à 80 °C,
b) ajouter l'ester de sorbitan au véhicule huileux chauffé, agiter de façon continue jusqu'à ce qu'une solution claire soit obtenue,
c) ajouter de la silice à la solution obtenue à l'étape b), agiter de façon continue jusqu'à ce qu'une solution claire soit obtenue,
d) refroidir à température ambiante la solution obtenue à l'étape c)
e) ajouter la base, le polyol et le probiotique à la solution refroidie, agiter le système de façon continue.
